# EUROPEAN PATENT APPLICATION

(11) **EP 4 613 297 A1**
(43) Date of publication of application: **10.09.2025**
(21) Application number: 23885073.9
(22) Date of filing: 03.11.2023
(51) Int. Cl.: A61M 5/14, A61M 5/00, A61M 39/00

(54) **INJECTION LINE SYSTEM FOR INJECTION DEVICES AND HIGH-PRESSURE RADIOGRAPHY INJECTION EQUIPMENT**

(30) Priority: 04.11.2022 CN 202222949790 U
(71) Applicant: Shenzhen Maiwei Biotech Co., Ltd., Shenzhen, Guangdong 518118 (CN)
(72) Inventor: CHEN, Zhiqiu, Shenzhen, Guangdong 518118 (CN); WEI, Naiwu, Shenzhen, Guangdong 518118 (CN)
(74) Representative: Manitz Finsterwald Patent- und Rechtsanwaltspartnerschaft mbB
(86) International application number: PCT/CN2023/129499
(87) International publication number: WO 2024/094148

(57) **Abstract**

Disclosed are an injection line system and a high-pressure contrast injection device. The injection line system comprises an injection line, a first liquid suction line, a second liquid suction line, a plurality of first puncture devices connected in series, and a second puncture device. The injection line is provided with a first liquid inlet connector, a second liquid inlet connector and an infusion connector, the first liquid inlet connector and the second liquid inlet connector are both connected with the infusion connector, one end of the first liquid suction line is connected with the first liquid inlet connector, one end of the second liquid suction line is connected with the second liquid inlet connector, the first puncture device closest to the first liquid suction line is connected with the other end of the first liquid suction line, and the second puncture device is connected with the other end of the second liquid suction line. The plurality of first puncture devices are arranged in series, so that other first liquid medicine containers can be replaced while ensuring that at least one first liquid medicine container provides liquid medicine normally, thereby improving the injection efficiency of the liquid medicine.

## Description

### TECHNICAL FIELD

The present disclosure relates to the technical field of medical devices, and in particular to an injection line system with puncture devices and a high-pressure contrast injection device.

### BACKGROUND

In the prior art, a high-pressure contrast injection device usually includes an injection pump, a syringe, and a line system, where the injection pump drives the syringe to move to draw and inject the liquid medicine. The line system is configured to connect the liquid medicine container to the syringe. With the widespread application of modern contrast-enhanced imaging technology, patients' demands for medical examinations are increasing. However, existing high-pressure contrast injection devices usually suffer from low injection efficiency. Therefore, it is necessary to provide a new high-pressure contrast injection device.

### SUMMARY OF THE INVENTION

A primary objective of the present disclosure is to provide an injection line system with puncture devices and a high-pressure contrast injection device capable of improving the efficiency of high-pressure contrast.

In order to achieve the above objective, the present disclosure provides an injection line system, including:
a plurality of first puncture devices connected in series, where two adjacent first puncture devices are connected in series via a first series connection line;
a first liquid suction line, where the first puncture device closest to the first liquid suction line is connected with one end of the first liquid suction line; and
an injection line, where the injection line is connected with the other end of the first liquid suction line.

In some embodiments, the injection line is provided with a first liquid inlet connector, a second liquid inlet connector, and an infusion connector, where the first liquid inlet connector and the second liquid inlet connector are both connected with the infusion connector, and the first liquid inlet connector is connected with the other end of the first liquid suction line; and the injection line system further includes a second liquid suction line and a second puncture device, one end of the second liquid suction line is connected with the second liquid inlet connector, and the other end of the second liquid suction line is connected with the second puncture device.

In some embodiments, the first liquid suction line is provided with a first controller for its opening/closing control, and the first series connection line is provided with a second controller for its opening/closing control.

In some embodiments, the injection line system further includes:
a third puncture device;
a third liquid suction line, where one end of the third liquid suction line is connected with the third puncture device, and the other end of the third liquid suction line is connected with the first liquid suction line; and
a third controller, where the third controller is arranged on the third liquid suction line for its opening/closing control.

In some embodiments, the first controller, the second controller, and the third controller are all liquid-stop clamps.

In some embodiments, the first liquid suction line includes a first liquid suction branch, a first multi-way connector, and a second liquid suction branch, where the first puncture device closest to the first liquid suction line is connected with the first liquid suction branch, a plurality of ports of the first multi-way connector are respectively connected with the first liquid suction branch, the second liquid suction branch, and the third liquid suction line, and the first liquid suction branch and the third liquid suction line are each provided with the liquid-stop clamp.

In some embodiments, the injection line includes a second multi-way connector, a first injection branch, a second injection branch, and a spiral infusion line, where a plurality of ports of the second multi-way connector are respectively connected with the first injection branch, the second injection branch, and the spiral infusion line, the first injection branch is connected with the first liquid inlet connector, the second injection branch is connected with the second liquid inlet connector, and the spiral infusion line is connected with the infusion connector.

In some embodiments, the first puncture device farthest from the first liquid suction line is provided with an air-inlet check valve.

In some embodiments, the injection line system further includes a third liquid suction line, a fourth controller, and a plurality of the third puncture devices connected in series, where two adjacent third puncture devices are connected in series via a second series connection line, and the fourth controller is arranged on the second series connection line for its opening/closing control; the first liquid suction line includes a first liquid suction branch, a second three-way connector, and a second liquid suction branch, where three ports of the second three-way connector are respectively connected with the third liquid suction line, the first liquid suction branch and the second liquid suction branch, the third liquid suction line is connected by puncture with one third puncture device, the second liquid suction branch is connected with the first liquid inlet connector, and the second three-way connector is provided with the first controller; the first controller has a first operating position and a second operating position; in the first operating position, the second liquid suction branch is communicated with the first liquid suction branch; and in the second operating position, the second liquid suction branch is communicated with the third liquid suction line.

The present disclosure also provides a high-pressure contrast injection device, including a control console, a first syringe, a second syringe, and the injection line system, where the first syringe and the second syringe are both connected with the control console, the first liquid inlet connector of the injection line system is connected with the first syringe, and the second liquid inlet connector of the injection line system is connected with the second syringe.

In some embodiments, the high-pressure contrast injection device further includes a first dual way check valve and a second dual way check valve, where the first dual way check valve and the second dual way check valve are each provided with a syringe port, a liquid suction port, and an infusion port, the syringe port of the first dual way check valve is connected with the first syringe, and the liquid suction port of the first dual way check valve is connected with the first liquid suction line; the syringe port of the second dual way check valve is connected with the second syringe, the liquid suction port of the second dual way check valve is connected with the second liquid suction line, and the infusion ports of the first dual way check valve and the second dual way check valve are both connected with the injection line; each of the dual way check valves a first operating state and a second operating state; in the first operating state, the infusion port is closed, such that the syringe port is communicated with the liquid suction port; and in the second operating state, the liquid suction port is closed, such that syringe port is communicated with the infusion port.

In some embodiments, the high-pressure contrast injection device further includes a stand body and a shelf, where the control console is disposed on the stand body, the shelf includes a shelf body, a container holder and a mounting latch, the mounting latch and the container holder are both in detachable connection with the shelf body, and the mounting latch is in detachable connection with the stand body.

In some embodiments, the detachable connection is implemented via a dovetail-shaped protrusion and a dovetail-shaped groove, where the dovetail-shaped protrusion mates with the dovetail-shaped groove.

A support stand used in conjunction with the injection line system, including a shelf, where the shelf includes a shelf body, a container holder and a mounting latch, the bottler holder and the mounting latch are both connected with the shelf body, the container holder is provided with a mounting portion for holding the first liquid medicine container, the mounting portion is provided with a base plate corresponding to a container stopper of the first liquid medicine container, and the base plate is configured to position the first puncture device and to keep the first puncture device in an upright state.

The base plate is provided with a positioning groove formed in a first direction and an insertion slot formed in a second direction, the positioning groove is communicated with the insertion slot, and the first direction is perpendicular to the second direction.

A high-pressure contrast injection device, including a support stand and the abovementioned injection line system, where the first puncture device of the injection line system can be connected by puncture with the first liquid medicine container, the support stand includes a stand body and a shelf, the shelf includes a shelf body, a mounting latch and a container holder, the mounting latch and the bottler holder are both connected with the shelf body, the mounting latch is connected with the stand body, the container holder is provided with a mounting portion for holding the first liquid medicine container, the mounting portion is provided with a base plate corresponding to a container stopper of the first liquid medicine container, and the base plate is configured to position the first puncture device and to keep the first puncture device in an upright state.

In some embodiments, the container holder and the mounting latch and the container holder are both in detachable connection with the shelf body, and the mounting latch is in detachable connection with the stand body.

In some embodiments, the shelf body is provided with a dovetail-shaped groove, the mounting latch and the bottle holder are each provided with a protrusion, and the protrusion mates with the groove.

In some embodiments, the mounting latch and the bottle holder are each provided with a dovetail-shaped groove, the shelf body is provided with a protrusion, and the protrusion mates with the groove.

In some embodiments, the base plate is provided with a positioning groove formed in a first direction and an insertion slot formed in a second direction, the positioning groove is communicated with the insertion slot, and the first direction is perpendicular to the second direction.

In some embodiments, the mounting portion further includes a slide plate, the base plate is disposed at a bottom of the side plate, the side plate and the bottom plate enclose a placement space, and a horizontal cross-section of the side plate is C-shaped.

In the technical solution, the plurality of first puncture devices are arranged in series, so that other first liquid medicine containers can be replaced while ensuring that at least one first liquid medicine container provides liquid medicine normally, thereby improving the injection efficiency of the liquid medicine.

### BRIEF DESCRIPTION OF THE DRAWINGS

In order to more clearly illustrate technical solutions in the embodiments of the present disclosure or in the prior art, a brief introduction to the accompanying drawings required for the description of the embodiments or the prior art will be provided below. Obviously, the accompanying drawings in the following description are only some of the embodiments of the present disclosure, and those ordinarily skilled in the art would also be able to derive other accompanying drawings from structures shown by these accompanying drawings without making creative efforts.
FIG. 1 is a schematic diagram of a three-dimensional structure of a high-pressure contrast injection device according to one embodiment of the present disclosure (before a control console is flipped).
FIG. 2 is a schematic diagram of a three-dimensional structure of a high-pressure contrast injection device according to one embodiment of the present disclosure (after a control console is flipped).
FIG. 3 is a schematic structural diagram from a top view of a support stand according to an embodiment of the present disclosure.
FIG. 4 is a schematic structural diagram of an injection line system according to one embodiment of the present disclosure.
FIG. 5 is a schematic structural diagram of an injection line system according to another embodiment of the present disclosure (when a first controller is in a first operating position).
FIG. 6 is a schematic structural diagram of an injection line system according to another embodiment of the present disclosure (when a first controller is in a second operating position).
FIG. 7 is a schematic structural diagram of an injection line system according to yet another embodiment of the present disclosure.
FIG. 8 is a schematic structural diagram of an injection line system according to still another embodiment of the present disclosure.
FIG. 9 is a schematic structural diagram of a shelf according to an embodiment of the present disclosure.
FIG. 10 is a schematic diagram of a three-dimensional structure of a high-pressure contrast injection device according to another embodiment of the present disclosure.
FIG. 11 is a partial enlarged view of Position P in FIG. 10.
FIG. 12 is a schematic structural diagram of the shelf in FIG. 10.
FIG. 13 is a schematic structural diagram of the support stand in FIG 10.

### DETAILED DESCRIPTION

The technical solutions in the embodiments of the present disclosure will be described clearly and completely with reference to the accompanying drawings in the embodiments of the present disclosure. It is obvious that the embodiments described are merely some embodiments rather than all embodiments of the present disclosure. On the basis of the embodiments in the present disclosure, all other embodiments acquired by those of ordinary skill in the art without making creative efforts fall within the scope of protection of the present disclosure.

It should be noted that all directional indications (such as upper, lower, left, right, front, back...) are involved in embodiments of the present disclosure are only used for explaining relative positional relations, motion conditions, etc. between components in a predetermined specific posture (as shown in the accompanying drawings), and if the specific posture is changed, the directional indications are correspondingly changed.

Furthermore, the terms "first", "second", and the like in the present disclosure are merely for the purpose of description, and cannot be construed as indicating or implying relative importance or implicitly specifying the number of technical features indicated. Thus, a feature defined with "first" and "second" may explicitly or implicitly include at least one of the features. In the description of the present disclosure, "a plurality of" means at least two, such as two, three, unless otherwise expressly and specifically defined.

In the present disclosure, unless expressly specified otherwise, the terms "connection/connected", "fixation/fixed", etc. are to be construed broadly, for example, "fixed" may be an unchangeable connection or a detachable connection, or integrated as a whole, may be a mechanical connection or an electrical connection, may be a directly connection or an indirect connection via an intermediary medium, or may be a communication between two elements or an interaction relation between two elements, unless expressly defined otherwise. Those of ordinary skill in the art can understand specific meanings of the above terms in the present disclosure according to specific circumstances.

In addition, the technical solutions of the embodiments in the present disclosure may be combined with one another, which must be based on the achievement by those of ordinary skill in the art, and when the combinations of the technical solutions contradict one another or cannot be achieved, it should be considered that the combinations of the technical solutions do not exist and do not fall within the scope of protection claimed in the present disclosure.

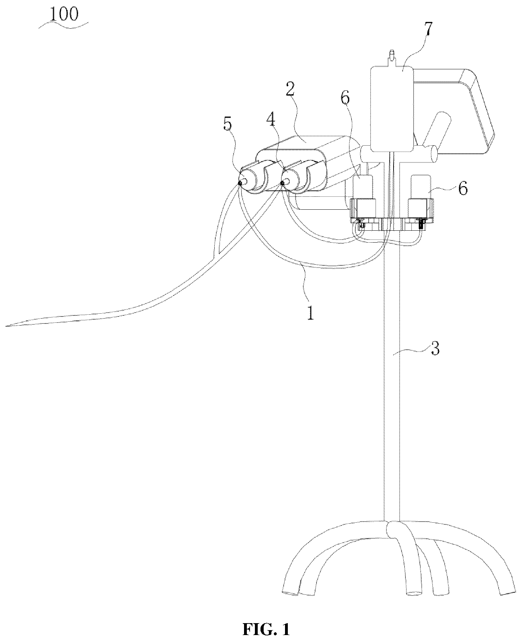

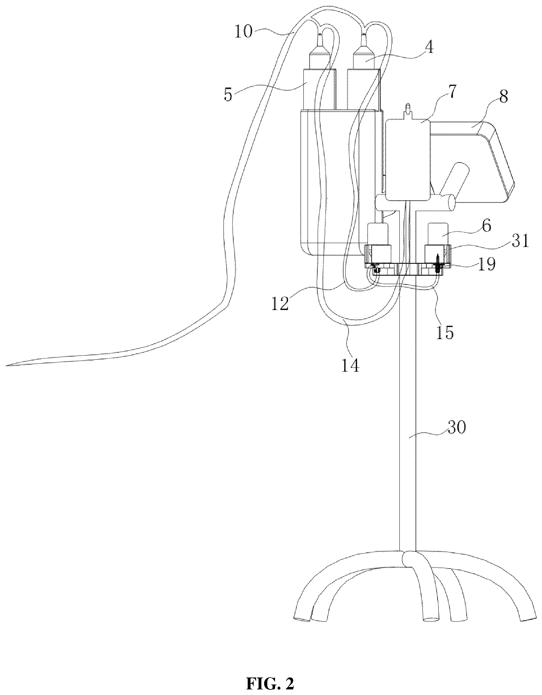

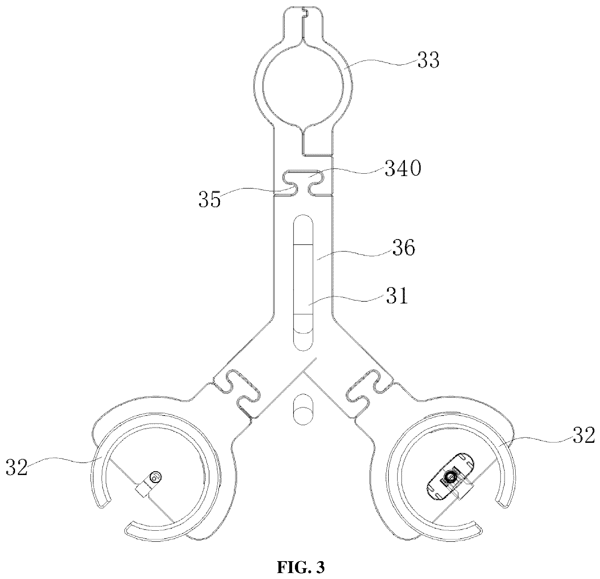

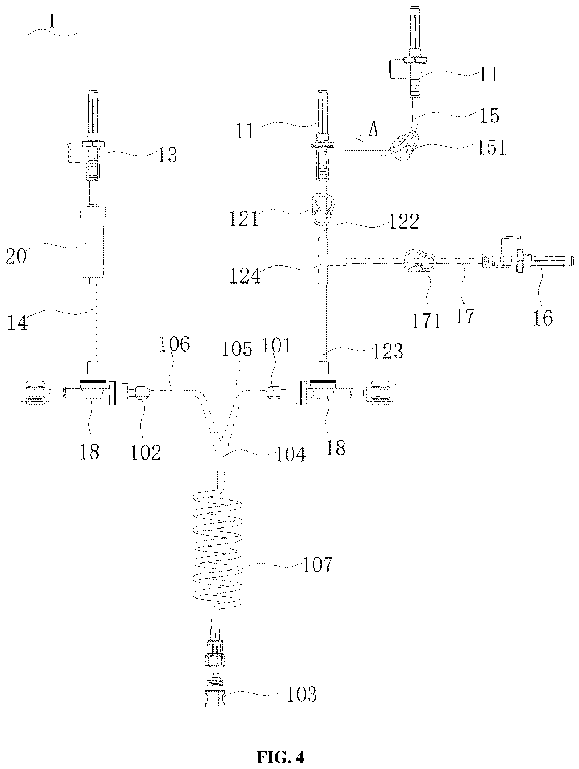

As shown in FIGs. 1-4, the injection line system 1 in this embodiment includes an injection line 10, a plurality of first puncture devices 11, a first liquid suction line 12, a second puncture device 13, and a second liquid suction line 14. The injection line 10 is provided with a first liquid inlet connector 101, a second liquid inlet connector 102, and an infusion connector 103, the first liquid inlet connector 101 is connected with the infusion connector 103 via a line, the second liquid inlet connector 102 is also connected with the infusion connector 103 via a line, and the infusion connector 103 can be connected with a patient end. Two adjacent first puncture devices 11 are connected in series via a first series connection line 15, such that the first puncture devices 11 are connected in series, and the first puncture devices 11 are connected in sequence in a flow direction A of liquid medicine. In the flow direction of the liquid medicine, one first puncture device 11 is closest to the first liquid suction line 12, and another first puncture device 11 is farthest from the first liquid suction line 12. The first liquid suction line 12 has two opposite ends, one end of the first liquid suction line is connected with the one first puncture device 11 that is closest to the first liquid suction line 12, and the other end thereof is connected with the first liquid inlet connector 101. The second liquid suction line 14 has two opposite ends, one end of the second liquid suction line is connected with the second puncture device 13, and the other end thereof is connected with the second liquid inlet connector 102.

The first puncture devices 11 and the second puncture device 13 are each provided with a puncture tip, and the puncture tip may be a sharp structure; and when being connected with a liquid medicine container with a container stopper, the puncture tip is capable of piercing the stopper of the liquid medicine container. When in use, each of the first puncture devices 11 is connected by puncture with the corresponding first liquid medicine container 6, the second puncture device 13 is connected by puncture with a second liquid medicine container 7, the first liquid inlet connector 101 may be connected with a first syringe 4, and the second liquid inlet connector 102 may be connected with a second syringe 5. When the first syringe 4 is working, liquid medicine can be drawn from the first liquid medicine container 6 via the first liquid suction line 12, first liquid medicine can be then injected via the injection line 10. When the second syringe 5 is working, liquid medicine can be drawn from the second liquid medicine container 7 via the second liquid suction line 14, second liquid medicine can be then injected via the injection line 10. The first liquid medicine and the second liquid medicine may be different; for example, the first liquid medicine may be a contrast agent, and the second liquid medicine may be saline. Of course, the second liquid medicine may also be liquid medicine that can be used in conjunction with the contrast agent.

Since the plurality of first puncture devices 11 are arranged, each of the first puncture devices 11 can be connected by puncture with the first liquid medicine container 6, such that the injection line system 1 can be simultaneously connected with a plurality of the first liquid medicine containers 6. The first liquid medicine containers 6 are connected in series via the first puncture devices 11, such that other first liquid medicine containers 6 can be replaced while ensuring that at least one first liquid medicine container 6 provides liquid medicine normally, thereby improving the injection efficiency of the liquid medicine.

For the injection line system, only one set of the first liquid medicine containers 6 may be arranged, which corresponds to one first liquid suction line 12. Alternatively, a plurality of sets of the first liquid medicine containers 6 may be provided, which correspond to a plurality of the first liquid suction lines 12, and each first liquid suction line 12 is connected with the injection line 10. Only one set of the second liquid medicine containers 7 may be arranged, which corresponds to one second liquid suction line 14. Alternatively, a plurality of sets of the second liquid medicine containers 7 may be provided, which correspond to a plurality of the second liquid suction lines 14, and each second liquid suction line 14 is connected with the injection line 10.

In some embodiments, the first liquid suction line 12 is provided with a first controller 121 for its opening/closing control. When the first controller 121 is opened, the first liquid suction line 12 allows liquid flow; when the first controller 121 is closed, the first liquid suction line 12 blocks liquid flow. The first series connection line 15 is provided with a second controller 151 for its opening/closing control. When the second controller 151 is opened, the first series connection line 15 allows liquid flow; and when the second controller 151 is closed, the first series connection line 15 blocks liquid flow.

When the first controller 121 is opened and the second controller 151 is closed, the liquid medicine in the first liquid medicine container 6 closest to the first liquid suction line 12 can flow to the first syringe 4 through the first liquid suction line 12. When both the first controller 121 and the second controller 151 are on, the first puncture devices 11 are connected in sequence. In the flow direction A of liquid medicine, the first liquid medicine in a subsequent first liquid medicine container 6 flows into a preceding first liquid medicine container 6 in the flow direction A of liquid medicine.

In some embodiments, the first puncture devices 11 may be two in number, and correspondingly, two first liquid medicine containers 6 may be provided. Each of the two first puncture devices 11 is connected by puncture with the corresponding first liquid medicine container 6. Referring to FIG. 1, the first liquid medicine container 6 near the first syringe 4 and located on a left side is capable of eliminating air bubbles in the first liquid medicine container 6 away from the first syringe 4 and located on a right side, and the liquid medicine in the first liquid medicine container 6 on the left side can be used as a safety reserve. The two first puncture devices 11 are connected via the first series connection line 15, and one of the first puncture devices 11 is directly connected with an end of the first liquid suction line 12. An aperture diameter of a liquid inlet hole of the first puncture device 11 away from the first liquid suction line 12 may be greater than or equal to an aperture diameter of a liquid inlet hole of the first puncture device 11 near the first liquid suction line 12, and the first series connection line 15 is connected with a liquid outlet hole of a subsequent first puncture device 11 and a liquid inlet hole of a preceding first puncture device 11. In some alternative structures, at least three first puncture devices 11 are provided, and correspondingly, at least three first liquid medicine containers 6 are provided, and each of the first puncture devices 11 is connected by puncture with the corresponding first liquid medicine container 6. The first puncture devices 11 are connected in series via the first series connection line 15, one of the first puncture devices 11 is directly connected with an end of the first liquid suction line 12, and one of the first puncture devices 11 is farthest from the first liquid suction line 12.

In some embodiments, a drip chamber 20 may be disposed between the second liquid suction line 14 and the second puncture device 13. The injection line 10 may include a first three-way connector 104, a first injection branch 105, a second injection branch 106, and a spiral infusion line 107, three ports of the first three-way connector 104 are respectively connected with the first injection branch 105, the second injection branch 106, and the spiral infusion line 107, the first injection branch 105 is connected with the first liquid inlet connector 101, the second injection branch 106 is connected with the second liquid inlet connector 102, and the spiral infusion line 107 is connected with the infusion connector 103. The first three-way connector 104 is a multi-way connector, the first three-way connector 104 may be Y-shaped, of course, the first three-way connector 104 may also be T-shaped, and a shape of the first three-way connector 104 is not limited herein. The spiral infusion line 107 is telescopically extendable. It can be extended when being pulled, and can be retracted when being released, thereby reducing a space occupied, and meeting the length requirements for clinical use.

In some embodiments, the injection line system 1 further includes a third puncture device 16 and a third liquid suction line 17. The third puncture device 16 is connected by puncture with a third liquid medicine container, third liquid suction line 17 has two opposing ends, one end is connected with the first liquid suction line 12, and the other end is connected with the third puncture device 16.

The first liquid suction line 12 may include a first liquid suction branch 122 and a second liquid suction branch 123, the first liquid suction branch 122 is connected with one of the first puncture devices 11, the second liquid suction branch 123 is connected with the first liquid inlet connector 101, and the first liquid suction branch 122, the second liquid suction branch 123, and the third liquid suction line 17 are connected via a second three-way connector 124. The third liquid suction line 17 may be provided with a third controller 171 its opening/closing control. When the third controller 171 is opened, the third liquid suction line 17 allows liquid flow, and the first syringe 4 can draw liquid medicine from the third liquid medicine container via the third liquid suction line 17; and when the third controller 171 is closed, the third liquid suction line 17 blocks liquid flow. In a specific structure, one end of the third liquid suction line 17 is connected with one port of the second three-way connector 124. The second three-way connector 124 is a multi-way connector, it may be Y-shaped or T-shaped, and a shape of the second three-way connector 124 is not limited herein.

The third puncture device 16 may be connected by puncture with the third liquid medicine container, and the third liquid medicine container be filled with a liquid medicine different from that of the first liquid medicine container 6. When different liquid medicine needs to be drawn, the first controller 121 may be off, and the third controller 171 may be on.

The first controller 121, the second controller 151, and the third controller 171 may be a liquid-stop clamp, which has an on state and an off state. When the first liquid medicine container 6 needs to be replaced, the corresponding second controller 151 may be off.

In some embodiments, the first liquid inlet connector 101 is connected with the first liquid suction line 12 via one dual way check valve 18, and the second liquid inlet connector 102 is connected with the second liquid suction line 14 via another dual way check valve 18. Each dual way check valve 18 may include a syringe port, a liquid suction port, and an infusion port. The syringe port is connected with the corresponding syringe, the liquid suction port is connected with the corresponding liquid suction line, and the infusion port is connected with the corresponding liquid inlet connector. For example, the syringe port of one of the dual way check valves 18 is connected with the first syringe 4, the liquid suction port thereof is connected with the first liquid suction line 12 (for example, the liquid suction port may be connected with the second liquid suction branch 123 of the first liquid suction line 12), and the infusion port thereof is connected with the first liquid inlet connector 101. The dual way check valve 18 has a first operating state and a second operating state. In the first operating state, the liquid suction port is opened while the infusion port is closed, and the syringe port communicated with the liquid suction port, such that the syringe is communicated with the liquid medicine container and draws the liquid medicine from the liquid medicine container. In the second operating state, the infusion port is opened while the liquid suction port is closed, and the syringe port is communicated with the infusion port, such that the syringe is communicated with and injection line 10 and delivers the liquid medicine to the patient end. A valve chamber of the dual way check valve 18 may be provided with a first diaphragm and a second diaphragm. When the first diaphragm is opened, while the second diaphragm is closed, the dual way check valve 18 is in the first operating state; and when the first diaphragm is closed, while the second diaphragm is opened, the dual way check valve 18 is in the second operating state. The dual way check valve may adopt an existing structure.

In some embodiments, the first puncture device 11 farthest from the first liquid suction line 12 is provided with an air-inlet check valve 19. During use, when the liquid medicine needs to be drawn, the air-inlet check valve 19 opens to allow air to enter the first puncture device 11; and when liquid medicine is not drawn, the air-inlet check valve 19 is closed. When at least three first puncture devices 11 are provided, the first puncture device 11 closest to the first liquid suction line 12 may not be provided with an air-inlet check valve 19, the first puncture device 11 farthest from the first liquid suction line 12 is provided with an air-inlet check valve 19, and the remaining first puncture devices 11 may not be provided with an air-inlet check valve 19. The second puncture device 13 may also be provided with an air-inlet check valve 19. The air-inlet check valve 19 may adopt an existing structure.

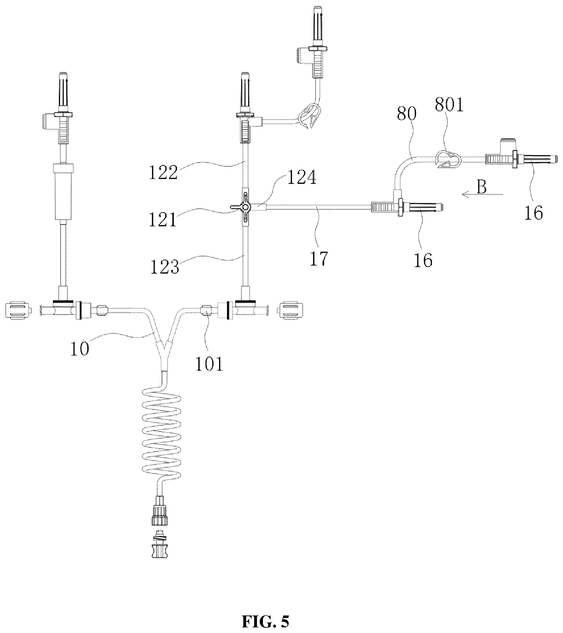

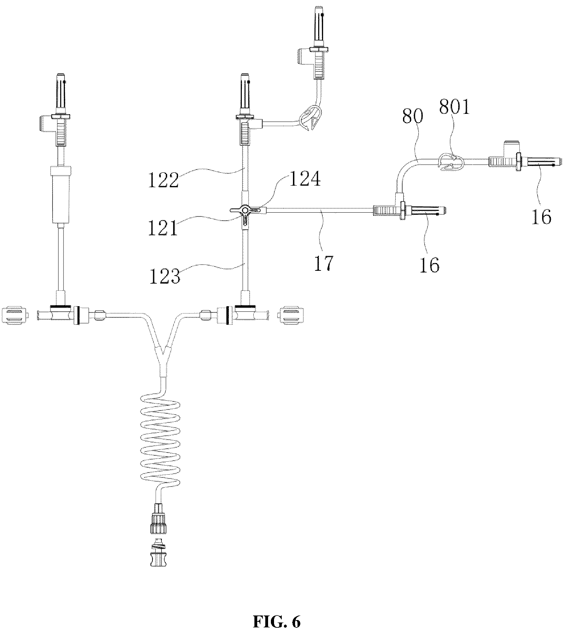

As shown in FIGs. 5 and 6, in some embodiments, the injection line system 1 further includes a third liquid suction line 17 and a plurality of the third puncture devices 16. Two adjacent third puncture devices 16 are connected in series via a second series connection line 80, such that the third puncture devices 16 are connected in series, and the third puncture devices 16 are connected in sequence in a flow direction B of liquid medicine. In the flow direction of liquid medicine, one third puncture device 16 is closest to the third liquid suction line 17, and another third puncture device 16 is farthest from the third liquid suction line 17. The second series connection line 80 is provided with a fourth controller 801 for its opening/closing control. The fourth controller 801 may be a liquid-stop clamp.

The third liquid suction line 17 has two opposite ends, one end of the third liquid suction line is connected with the one third puncture device 16 that is closest to the third liquid suction line 17, and the other end thereof is connected with the first liquid inlet connector 101.

The first liquid suction line 12 may include a first liquid suction branch 122 and a second liquid suction branch 123, the first liquid suction branch 122 is connected with one of the first puncture devices 11, the second liquid suction branch 123 is connected with the first liquid inlet connector 101, and the first liquid suction branch 122, the second liquid suction branch 123, and the third liquid suction line 17 are connected via a second three-way connector 124.

The second three-way connector 124 is provided with a first controller 121 for its opening/closing control, and the first controller 121 has a first operating position and a second operating position. In the first operating position, the first liquid suction branch 122 is communicated with the second liquid suction branch 123, the first liquid suction branch 122 and the second liquid suction branch 123 are disconnected from the third liquid suction line 17, and the first liquid medicine in the first liquid medicine container 6 can flow to the injection line 10 through the first suction line 12. In the second operating position, the third liquid suction line 17 is communicated with the second liquid suction branch 123, the second liquid suction branch 123 and the third liquid suction line 17 are both disconnected from the first liquid suction branch 122, and the third liquid medicine in the third liquid medicine container can flow to the injection line 10 through the second liquid suction branch 123.

The third liquid medicine container supplies the third liquid medicine, the third liquid medicine may be different from the first liquid medicine supplied by the first liquid medicine container. For example, the first liquid medicine and the third liquid medicine may be two different contrast agents. When a conventional first liquid medicine is drawn, the first controller 121 may be in the first operating position, as shown in FIG. 5. When a special third liquid medicine is drawn, the first controller 121 may be in the second operating position, as shown in FIG. 6.

By arranging a plurality of the third puncture devices 16 and the third liquid medicine containers connected in series, the liquid medicine in the preceding third liquid medicine container can be drawn after the liquid medicine in the subsequent third liquid medicine container is drawn in the flow direction B of liquid medicine.

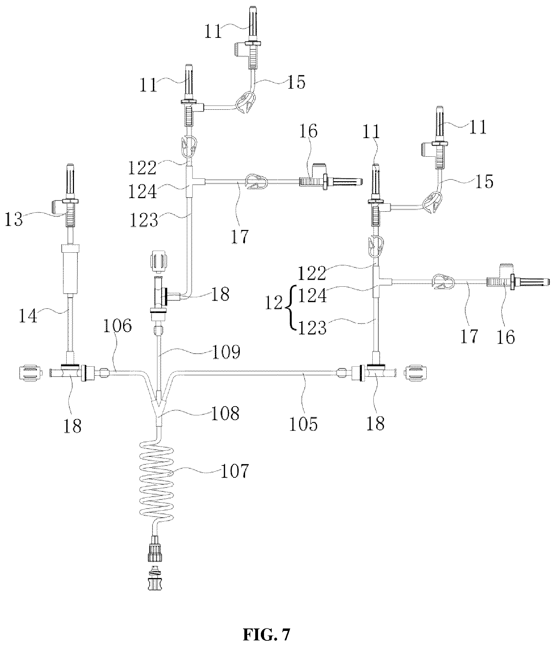

As shown in FIG. 7, in some embodiments, the injection line system 1 includes an injection line 10, two sets of first puncture devices 11, two first liquid suction line 12, a second puncture device 13, and a second liquid suction line 14. Each set of the first puncture devices 11 corresponds to one first liquid suction line 12, and each set includes a plurality of the first puncture devices 11 connected in series via a first series connection line 15. The first puncture devices 11, after being connected in series, are connected with the injection line 10 via the first liquid suction line 12, and the second puncture device 13 is connected with the injection line 10 via the second liquid suction line 14.

The abovementioned injection line system 1 further includes two sets of third puncture devices 16 and two third liquid suction lines 17, the two sets of third puncture devices 16 are respectively connected with the two third liquid suction lines 17, and the two third liquid suction lines 17 are respectively connected with the two first liquid suction lines 12.

Specifically, the injection line 10 may include a four-way connector 108, a first injection branch 105, a second injection branch 106, a spiral infusion line 107, and a third injection branch 109. Four ports of the four-way connector 108 are respectively connected with the first injection branch 105, the second injection branch 106, the third injection branch 109, and the spiral infusion line 107. Each first liquid suction line 12 includes a first liquid suction branch 122 and a second liquid suction branch 123, and the first liquid suction branch 122, the second liquid suction branch 123, and the corresponding third liquid suction line 17 are connected via a second three-way connector 124. The two second liquid suction branches 123 are respectively connected with the first injection branch 105 and the third injection branch 109 via dual way check valves 18. The second injection branch 106 is connected with the second liquid suction line 14 via another dual way check valve 18. When the liquid medicine is drawn, the syringe port of the dual way check valve 18 is communicated with the liquid suction port, such that the first syringe 4 and the second syringe 5 can draw liquid medicine from the corresponding first liquid medicine container 6 and the second liquid medicine container 7, respectively. When the liquid medicine is injected, the infusion port and the syringe port of the dual way check valve 18 is communicated with each other, and the first syringe 4 and the second syringe 5 can inject the liquid medicine to the patient end through the spiral infusion line 107.

Each of the first liquid suction lines 12 and the third liquid suction lines 17 is provided with a controller for its opening/closing control, the controller may be a liquid-stop clamp, for example, the liquid-stop clamp is arranged on each of the first series connection lines 15, each of the first liquid suction branch 122, and each of the third liquid suction line 17. The second puncture device 13 may be connected by puncture with the second liquid medicine container, the first puncture device 11 may be connected by puncture with the first liquid medicine container, the third puncture device 16 may be connected by puncture with the third liquid medicine container, and the first liquid medicine container and the third liquid medicine container may be filled with different liquid medicines. The different liquid medicine can be drawn by controlling opening/closing of different controllers.

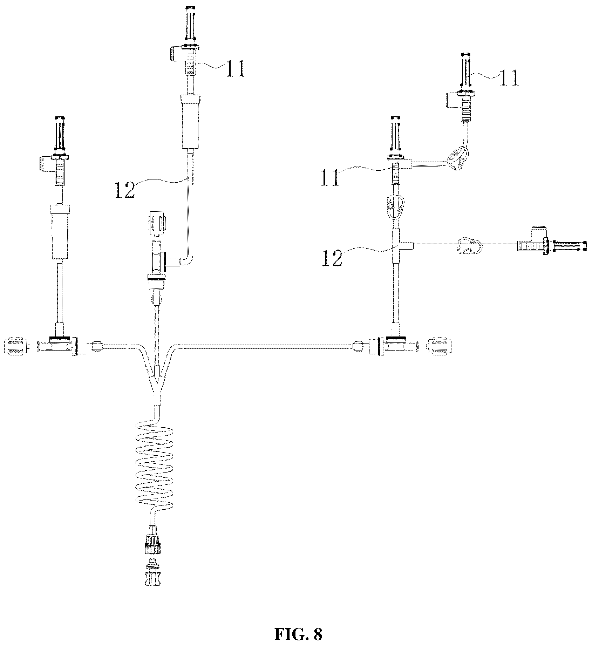

The injection line system 1 may include one first liquid suction line 12, or two, three, or more first liquid suction lines 12, and a specific number may be determined according to design, manufacturing, and operational requirements. When a plurality of the first liquid suction lines 12 are provided, at least one of the first liquid suction lines 12 is connected with a plurality of first puncture devices 11 in series, that is, each of the first liquid suction line 12 may be connected with the plurality of first puncture devices 11 in series, or only some of the first liquid suction lines 12 may be connected with the plurality of first puncture devices 11 in series. For example, as shown in FIG. 8, one first liquid suction line 12 is connected with the plurality of first puncture devices 11 in series, while another first liquid suction line 12 is connected with a single first puncture device 11. When the plurality of the first liquid suction lines 12 are provided, one or more of the first liquid suction lines 12 may be connected with the third liquid suction line 17, or all of them may be connected with the third liquid suction line 17, or none of them may be connected with the third liquid suction line 17.

As shown in FIGs. 1-4, a high-pressure contrast injection device 100 includes a control console 2, a first syringe 4, a second syringe 5, and the abovementioned injection line system 1. The first syringe 4 is configured to draw liquid medicine from a corresponding liquid medicine container and inject the liquid medicine to the patient end. The second syringe 5 is configured to draw liquid medicine from a corresponding liquid medicine container and inject the liquid medicine to the patient end. The injection line system 1 includes a first liquid inlet connector 101 and a second liquid inlet connector 102, the first syringe 4 is connected with both the first liquid inlet connector 101 and the first liquid suction line 12, and the first syringe 4 is configured to draw liquid medicine through the first liquid suction line 12 and inject the liquid medicine through the first liquid inlet connector 101. The second syringe 5 is connected with both the second liquid inlet connector 102 and the second liquid suction line 14, and the second syringe 5 is configured to draw liquid medicine through the second liquid suction line 14 and inject the liquid medicine through the second liquid inlet connector 102.

The injection line system 1 may further include dual way check valves 18, the first syringe 4 may be connected with a syringe port of one dual way check valve 18, an infusion port of the one dual way check valve 18 may be connected with the first liquid inlet connector 101, and a liquid suction port of the one dual way check valve 18 may be connected with the first liquid suction line 12. The second syringe 5 may be connected with a syringe port of another dual way check valve 18, an infusion port of the another dual way check valve 18 may be connected with the second liquid inlet connector 102, and a liquid suction port of the another dual way check valve 18 may be connected with the second liquid suction line 14.

The high-pressure contrast injection device 100 may further include a support stand 3, the support stand 3 is configured to carry the control console 2, the first syringe 4, the second syringe 5, and the injection line system 1. The support stand 3 includes a stand body 30 and a shelf 31 mounted on the stand body 30. The stand body 30 has a predetermined height and may be placed upright on a ground, and the control console 2 may be fixedly mounted on the stand body 30. The shelf 31 includes a shelf body 36, and a mounting latch 33 and two container holders 32 arranged on the shelf body 36, the mounting latch 33 is configured to mount the shelf 31 to the stand body 30, and the container holders 32 are configured to hold the liquid medicine containers. For example, the container holders 32 may be configured to hold the first liquid medicine container 6 containing a contrast agent. In order to facilitate the mounting and removal of the shelf 31 and the liquid medicine containers, both the mounting latch 33 and the container holders 32 are in detachable connection with the shelf body 36 via connecting assemblies. For example, each connecting assembly includes a dovetail-shaped groove 340 formed on the shelf body 36 and a dovetail-shaped protrusion 35 mated with the dovetail-shaped groove 340. The dovetail-shaped protrusion 35 is arranged on the container holders 32 and the mounting latch 33, such that a detachable connection is achieved through the cooperation between the dovetail-shaped protrusion and the dovetail-shaped groove. Of course, the dovetail-shaped protrusion 35 may be arranged on the shelf body 36, and the dovetail-shaped groove 340 may be formed on the container holder 32 and the mounting latch 33. Since the container holders 32 and the mounting latch 33 are in detachable connection with the shelf body 36, different models of the container holders 32 and mounting latch 33 may be replaced, such that the shelf 31 can match the stand body 30 of different sizes and the liquid medicine containers of different sizes.

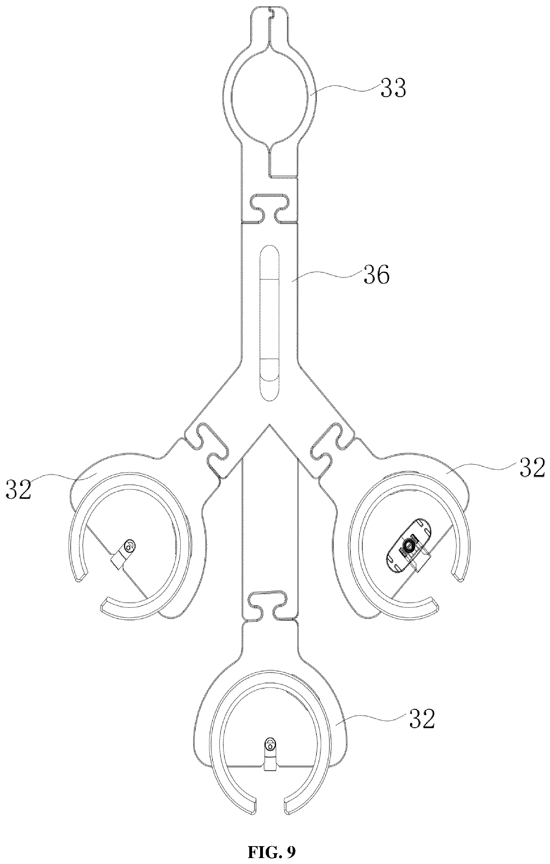

As shown in FIGs. 8 and 9, in some embodiments, the shelf 31 may include the mounting latch 33 and at least three container holders 32, such that the shelf 31 can hold at least three liquid medicine containers. A number of container holders 32 may be determined according to design, manufacturing, and usage requirements. The mounting latch 33 and the container holders 32 are detachably fixed to the shelf body 36 by means of latch connection, plug-in connection, or screw connection. The mounting latch 33 may include two portions capable of being on and off relative to each other. When being off, the mounting latch 33 can be tightly sleeved on the stand body 30. When being on, the mounting latch 33 can be removed from the stand body 30.

The high-pressure contrast injection device 100 may further include an operation panel 8, and the operation panel 8 is mounted on the stand body 30. In one specific structure, the control console 2 may be rotatably connected with the stand body 30, such that it can be flipped within a predetermined angular range. In another specific structure, the control console 2 may include an injection pump.

When in use, the control console 2 drives the first syringe 4 to draw the first liquid medicine from the first liquid medicine container 6, and drives the second syringe 5 to draw the second liquid medicine from the second liquid medicine container 7. Then, the control console 2 may drive the first syringe 4 to inject the first liquid medicine into the injection line 10, and drive the second syringe 5 to inject the second liquid medicine into the injection line 10, and the first liquid medicine and the second liquid medicine are mixed in the spiral infusion line 107 and are then delivered to the patient end through the infusion connector 103.

In the high-pressure contrast injection device 100, since the support stand 3 is provided, the first puncture device 11 and the second puncture device 13 of the first puncture device can be vertically inserted when the liquid medicine container or the saline container is placed, thereby reducing the difficulties caused by clinical piercing operations. By providing the first controller 121, the second controller 151, and the third controller 171, it is possible to draw the liquid medicine from different liquid medicine containers. By providing the dual one-way valves 18, the first liquid medicine can be prevented from flowing into the second liquid suction line 14, and the second liquid medicine can be prevented from flowing into the first liquid suction line 12. By arranging a plurality of the first puncture devices 11 and the first liquid medicine containers 6 connected in series, the liquid medicine in the preceding first liquid medicine container 6 can be drawn after the liquid medicine in the subsequent first liquid medicine container 6 is drawn in the flow direction A of liquid medicine. By causing the container holders 32 and the mounting latch 33 to be in detachable connection with the stand body 30, the system can be adapted to the liquid medicine containers of different sizes and to the control consoles 2 of different models.

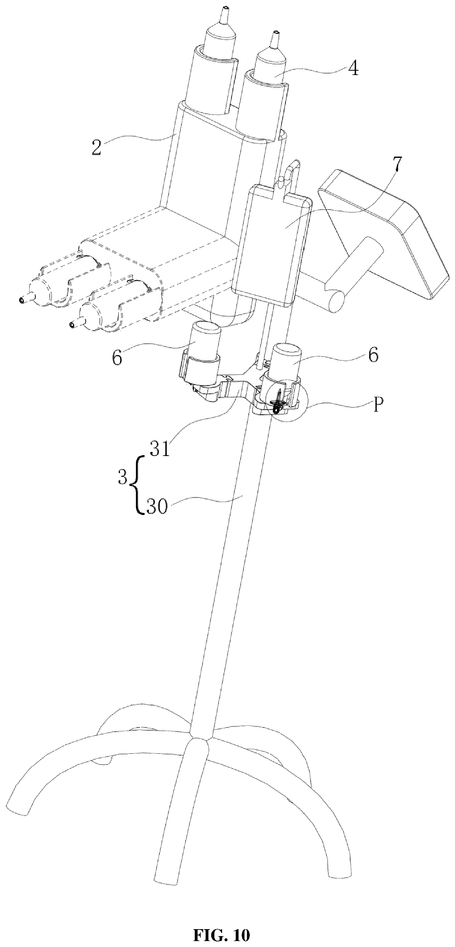

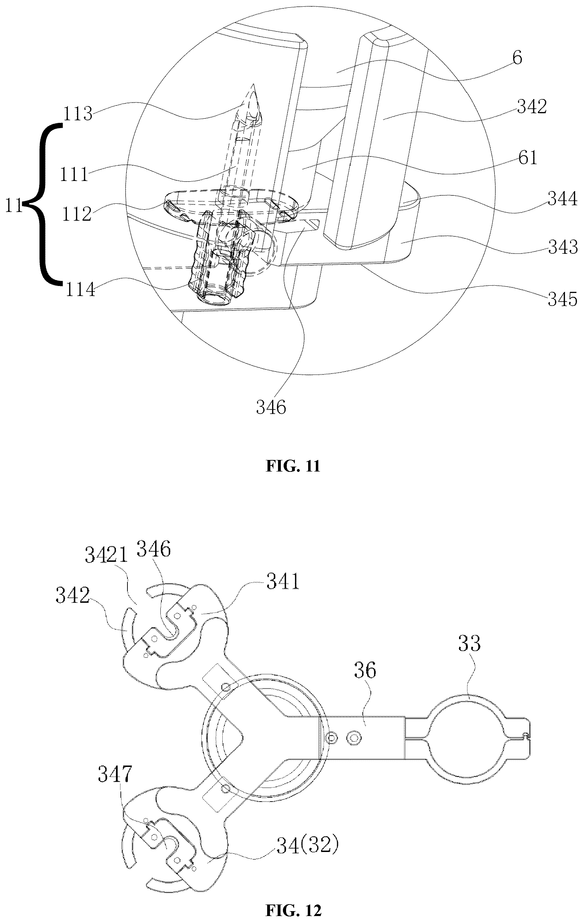

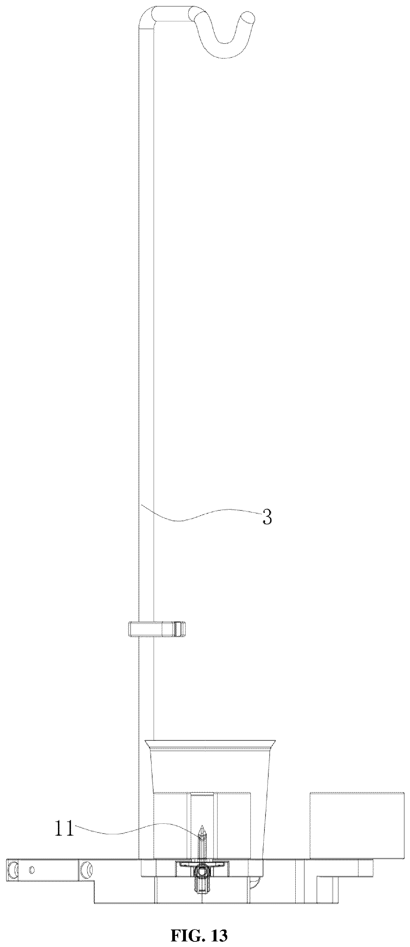

As shown in FIGs. 10-13, in some embodiments, the high-pressure contrast injection device includes a control console 2, a first syringe 4, and the abovementioned injection line system. The first syringe 4 is configured to draw liquid medicine from the corresponding first liquid medicine container 6 and inject the liquid medicine to the patient end. The first liquid medicine container 6 is connected by puncture with the first puncture device 11. The high-pressure contrast injection device may further include a support stand 3, the support stand 3 includes a stand body 30 and a shelf 31 mounted on the stand body 30, and the stand body 30 has a predetermined height and may be placed upright on a ground. The shelf 31 includes a shelf body 36, and a mounting latch 33 and container holders 32 arranged on the shelf body 36, and the mounting latch 33 can be connected with the stand body 30. Each container holder 32 is provided with a mounting portion 34 for holding the first liquid medicine container 6, the mounting portion 34 is provided with a base plate 341 corresponding to a container stopper 61 of the first liquid medicine container 6, and the base plate 341 is configured to position the first puncture device 11 and to keep the first puncture device 11 in an upright state, such that when the first liquid medicine container 6 is placed and positioned on the mounting portion 34, a puncture tip 113 of the first puncture device 11 can puncture through the container stopper 61 of the first liquid medicine container 6.

The mounting portion 34 may further include a side plate 342, the side plate 342 is vertically arranged relative to the base plate 341, the base plate 341 is disposed at a bottom of the side plate 342, and a placement space for placing the first liquid medicine container 6 is formed between the side plate 342 and the base plate 341. A lateral opening 3421 is formed on the side plate 342, such that a cross section of the side plate 342 in a first direction is C-shaped, and the first direction may be a horizontal direction, that is, a horizontal cross-section of the side plate 342 is C-shaped. The base plate 341 includes a side surface 343, as well as an upper surface 344 and a lower surface 345 arranged opposite each other in a second direction. The side surface 343 is located between the upper surface 344 and the lower surface 345, and the second direction may be a vertical direction. A positioning groove 346 is formed by recessing from side surface 343 in the first direction, an insertion slot 347 is formed by vertically penetrating the upper surface 344 and the lower surface 345 in the second direction, and the insertion slot 347 is communicated with the positioning groove 346. The first puncture device 11 may include a puncture device body 111 with a relatively small outer diameter and a wing portion 112 that extends radially outward from an outer wall of the puncture device body 111, such that an outer diameter of the wing portion 112 is greater than that of the puncture device body 111. Two ends of the puncture device body 111 are respectively the puncture tip 113 and a connecting tail end 114, and the connecting tail end 114 can be connected with the line. The positioning groove 346 is adapted to the wing portion 112, and the puncture device body 111 is adapted to the insertion slot 347. When in use, the first puncture device 11 is moved in the first direction, such that the wing portion 112, having a greater outer diameter, of the first puncture device 11, is embedded in the positioning groove 346, the puncture tip 113 of the first puncture device 11 extends upward beyond the upper surface 344, thereby allowing the first puncture device 11 to be installed and positioned on the base plate 341. When the first liquid medicine container 6 is mounted in the mounting portion 34, the puncture tip 113 punctures the container stopper 61 of the first liquid medicine container 6, thereby realizing communication between the first liquid medicine container 6 and the first puncture device 11. By pre-positioning the first puncture device 11 on the container holder 32, the first liquid medicine container 6 is connected by puncture with the first puncture device 11 when it is mounted in place.

The injection line system may include a plurality of first puncture devices 11, first liquid suction lines 12, and injection lines 10, the first puncture devices 11 are connected in series via the first series connection line 15, the first liquid suction lines 12 are respectively connected with the first puncture devices 11 and the injection lines 10, and the injection lines 10 can be in turn connected with the patient end. The injection line system may further include a second liquid suction line 14 and a second puncture device 13, and the second liquid suction line 14 is connected with the second puncture device 13 to the injection line 10. A plurality of the second puncture devices 13 may also be provided, and the second puncture devices 13 can be connected in series via the series connection lines. The first liquid suction line 12 and/or the second liquid suction line 14 may further be connected with the third liquid suction line 17, the third liquid suction line 17 may be connected with the third puncture device 16 and the corresponding first liquid suction line 12 or the second liquid suction line 14. When a single second puncture device 13 is provided, it may be provided with an air-inlet check valve 19. When a plurality of the second puncture devices 13 connected in parallel are provided, the second puncture device 13 farthest from the second liquid inlet line 14 may be provided with the air-inlet check valve.

The support stand 3 includes a shelf 31, the shelf 31 includes a shelf body 36, and a mounting latch 33 and a container holder 32, the mounting latch 33 is connected with an upright stand body 30, and the container holder 32 is configured to hold the first liquid medicine container 6. The container holder 32 is provided with a base plate 341, and the base plate 341 is disposed corresponding to the container stopper 61 of the first liquid medicine container 6. When the first liquid medicine container 6 is placed on the container holder 32, the container stopper 61 may be attached to the base plate 341 or be spaced from the base plate 341 by a predetermined gap. The first liquid medicine container 6 may be filled with a contrast agent, a saline solution, or other liquid medicine that can be used for the injection of contrast agent. A plurality of the container holders 32 may be provided, all of them may be configured to hold the first liquid medicine containers 6, or to hold the second liquid medicine containers 7; or some of them are configured to hold the first liquid medicine containers 6 and the remaining ones are configured to hold the second liquid medicine containers 7. The first liquid medicine container 6 may be filled with the first liquid medicine, the second liquid medicine container 7 may be filled with the second liquid medicine, and the first liquid medicine and the second liquid medicine may be the same or different.

What is described above is only a preferred embodiment of the present disclosure and does not limit the patent scope of the present disclosure, and equivalent structure change made by utilizing contents of the description and the drawings in the present disclosure and used directly or indirectly in other related technical fields shall all fall within the scope of protection of the present disclosure in a similar way.

## Claims

1. An injection line system, **characterized by** comprising:
a plurality of first puncture devices connected in series, **characterized in that** two adjacent first puncture devices are connected in series via a first series connection line;
a first liquid suction line, **characterized in that** the first puncture device closest to the first liquid suction line is connected with one end of the first liquid suction line; and
an injection line, **characterized in that** the injection line is connected with the other end of the first liquid suction line.

2. The injection line system according to claim 1, **characterized in that** the injection line is provided with a first liquid inlet connector, a second liquid inlet connector, and an infusion connector, the first liquid inlet connector and the second liquid inlet connector are both connected with the infusion connector, and the first liquid inlet connector is connected with the other end of the first liquid suction line; and the injection line system further comprises:
a second liquid suction line, **characterized in that** one end of the second liquid suction line is connected with the second liquid inlet connector, and
a second puncture device, **characterized in that** the second puncture device is connected with the other end of the second liquid suction line.

3. The injection line system according to claim 2, **characterized in that** the first liquid suction line is provided with a first controller for opening/closing control of the first liquid suction line, and the first series connection line is provided with a second controller for opening/closing control of the first series connection line.

4. The injection line system according to claim 3, **characterized by** further comprising:
a third puncture device;
a third liquid suction line, **characterized in that** one end of the third liquid suction line is connected with the third puncture device, and the other end of the third liquid suction line is connected with the first liquid suction line; and
a third controller, **characterized in that** the third controller is arranged on the third liquid suction line for opening/closing control of the third liquid suction line.

5. The injection line system according to claim 4, **characterized in that** the first controller, the second controller, and the third controller are all liquid-stop clamps.

6. The injection line system according to claim 4, **characterized in that** the first liquid suction line comprises a first liquid suction branch, a first multi-way connector, and a second liquid suction branch, the first puncture device closest to the first liquid suction line is connected with the first liquid suction branch, a plurality of ports of the first multi-way connector are respectively connected with the first liquid suction branch, the second liquid suction branch, and the third liquid suction line, and the first liquid suction branch and the third liquid suction line are each provided with the liquid-stop clamp.

7. The injection line system according to claim 3, **characterized in that** the injection line comprises a second multi-way connector, a first injection branch, a second injection branch, and a spiral infusion line, a plurality of ports of the second multi-way connector are respectively connected with the first injection branch, the second injection branch, and the spiral infusion line, the first injection branch is connected with the first liquid inlet connector, the second injection branch is connected with the second liquid inlet connector, and the spiral infusion line is connected with the infusion connector.

8. The injection line system according to claim 1, **characterized in that** the first puncture device farthest from the first liquid suction line is provided with an air-inlet check valve.

9. The injection line system according to claim 4, **characterized by** further comprising a third liquid suction line, a fourth controller, and a plurality of the third puncture devices connected in series, **characterized in that** two adjacent third puncture devices are connected in series via a second series connection line, and the fourth controller is arranged on the second series connection line for opening/closing control of the second series connection line; the first liquid suction line comprises a first liquid suction branch, a second three-way connector, and a second liquid suction branch, three ports of the second three-way connector are respectively connected with the third liquid suction line, the first liquid suction branch and the second liquid suction branch, the third liquid suction line is connected by puncture with one third puncture device, the second liquid suction branch is connected with the first liquid inlet connector, and the second three-way connector is provided with the first controller; the first controller has a first operating position and a second operating position; in the first operating position, the second liquid suction branch is communicated with the first liquid suction branch; and in the second operating position, the second liquid suction branch is communicated with the third liquid suction line.

10. A high-pressure contrast injection device, **characterized by** comprising a control console, a first syringe, a second syringe, and the injection line system according to any one of claims 2-9, **characterized in that** the first syringe and the second syringe are both connected with the control console, the first liquid inlet connector of the injection line system is connected with the first syringe, and a second liquid inlet connector of the injection line system is connected with the second syringe.

11. The high-pressure contrast injection device according to claim 10, **characterized by** further comprising a first dual way check valve and a second dual way check valve, **characterized in that** the first dual way check valve and the second dual way check valve are each provided with a syringe port, a liquid suction port, and an infusion port, the syringe port of the first dual way check valve is connected with the first syringe, and the liquid suction port of the first dual way check valve is connected with the first liquid suction line; the syringe port of the second dual way check valve is connected with the second syringe, the liquid suction port of the second dual way check valve is connected with the second liquid suction line, and the infusion ports of the first dual way check valve and the second dual way check valve are both connected with the injection line; the first dual way check valve and the second dual way check valve each have a first operating state and a second operating state; in the first operating state, the infusion port is closed, and the syringe port is communicated with the liquid suction port; and in the second operating state, the liquid suction port is closed, and the syringe port is communicated with the infusion port.

12. The high-pressure contrast injection device according to claim 10, **characterized by** further comprising a stand body and a shelf, **characterized in that** the control console is disposed on the stand body, the shelf comprises a shelf body, a container holder and a mounting latch, the mounting latch and the container holder are both in detachable connection with the shelf body, and the mounting latch is in detachable connection with the stand body.

13. A support stand used in conjunction with the injection line system according to any one of claims 1-9, **characterized by** comprising a shelf, **characterized in that** the shelf comprises a shelf body, a container holder and a mounting latch, the bottler holder and the mounting latch are both connected with the shelf body, the container holder is provided with a mounting portion for holding a first liquid medicine container, the mounting portion is provided with a base plate corresponding to a container stopper of the first liquid medicine container, and the base plate is configured to position the first puncture device and to keep the first puncture device in an upright state.

14. The support stand according to claim 13, **characterized in that** the base plate is provided with a positioning groove formed in a first direction and an insertion slot formed in a second direction, the positioning groove is communicated with the insertion slot, and the first direction is perpendicular to the second direction.

15. A high-pressure contrast injection device, **characterized by** comprising a support stand and the injection line system according to any one of claims 1-9, **characterized in that** a first puncture device of the injection line system is connected by puncture with a first liquid medicine container, the support stand comprises a stand body and a shelf, the shelf comprises a shelf body, a mounting latch and a container holder, the mounting latch and the bottler holder are both connected with the shelf body, the mounting latch is connected with the stand body, the container holder is provided with a mounting portion for holding the first liquid medicine container, the mounting portion is provided with a base plate corresponding to a container stopper of the first liquid medicine container, and the base plate is configured to position the first puncture device and to keep the first puncture device in an upright state.

16. The high-pressure contrast injection device according to claim 15, **characterized in that** the container holder and the mounting latch and the container holder are both in detachable connection with the shelf body, and the mounting latch is in detachable connection with the stand body.

17. The high-pressure contrast injection device according to claim 16, **characterized in that** the shelf body is provided with a dovetail-shaped groove, the mounting latch and the bottle holder are each provided with a protrusion, and the protrusion mates with the dovetail-shaped groove.

18. The high-pressure contrast injection device according to claim 16, **characterized in that** the mounting latch and the bottle holder are each provided with a dovetail-shaped groove, the shelf body is provided with a protrusion, and the protrusion mates with the dovetail-shaped groove.

19. The high-pressure contrast injection device according to claim 15, **characterized in that** the base plate is provided with a positioning groove formed in a first direction and an insertion slot formed in a second direction, the positioning groove is communicated with the insertion slot, and the first direction is perpendicular to the second direction.

20. The high-pressure contrast injection device according to claim 19, **characterized in that** the mounting portion further comprises a slide plate, the base plate is disposed at a bottom of the side plate, the side plate and the bottom plate enclose a placement space, and a horizontal cross-section of the side plate is C-shaped.
